(19) Europäisches Patentamt
European Patent Office
Office européen des brevets

(11) EP 1 210 931 B1

(12) **FASCICULE DE BREVET EUROPEEN**

(45) Date de publication et mention de la délivrance du brevet:
**05.04.2006 Bulletin 2006/14**

(51) Int Cl.:
*A61K 8/27* (2006.01) *A61K 8/19* (2006.01)
*A61K 8/34* (2006.01) *A61Q 5/10* (2006.01)

(21) Numéro de dépôt: **01403101.7**

(22) Date de dépôt: **03.12.2001**

(54) **Composition de coloration des fibres keratiniques**

Keratinisches Faserfärbemittel

Keratinic fibres dyeing composition

(84) Etats contractants désignés:
**AT BE CH CY DE DK ES FI FR GB GR IE IT LI LU MC NL PT SE TR**

(30) Priorité: **04.12.2000 FR 0015695**

(43) Date de publication de la demande:
**05.06.2002 Bulletin 2002/23**

(73) Titulaire: **L'OREAL**
**75008 Paris (FR)**

(72) Inventeur: **Pruche, Francis**
**60300 Senlis (FR)**

(74) Mandataire: **Catherine, Alain et al**
**Cabinet Harlé & Phélip**
**7, rue de Madrid**
**75008 Paris (FR)**

(56) Documents cités:
**EP-A- 0 621 029** **EP-A- 0 642 783**
**DE-A- 2 222 001** **DE-A- 19 859 682**
**FR-A- 2 748 274** **GB-A- 2 307 175**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une composition de coloration des fibres kératiniques, un procédé d'obtention d'une telle composition de coloration et son utilisation pour la coloration de fibres kératiniques.

Plus particulièrement, la présente invention concerne une composition de coloration du cheveu qui, après application sur celui-ci, donne une nuance de couleur originale, et permet d'augmenter la tenue de la couleur sur les cheveux.

Dans le domaine de la coloration des fibres kératiniques telles que les cheveux, les cils, les sourcils et les poils, il est connu d'utiliser des compositions tinctoriales contenant des bases d'oxydation, tels que les p-phénylènes diamines et les ortho- ou para-aminophénols, pour teindre les fibres kératiniques, en particulier les cheveux humains.

On sait également, qu'on peut faire varier les nuances obtenues avec ces bases d'oxydation en les associant à des coupleurs, encore appelés modificateurs de coloration, tels que les métadiamines aromatiques, les méta-aminophénols, les métadiphénols et les dérivés de dihydroxyindole.

La révélation de la coloration de ces compositions nécessite l'emploi d'un agent oxydant choisi par exemple parmi le peroxyde d'hydrogène, le peroxyde d'urée, les bromates de métaux alcalins, les persels, tels que les perborates et les persulfates, le peroxyde d'hydrogène étant particulièrement préféré.

On utilise aussi des catalyseurs enzymatiques pour activer la coloration de précurseurs de coloration. Ainsi, on active la coloration de polyphénols par oxydation en présence de polyphénoloxydase naturelle. A titre d'exemple, de la catéchine, en présence de polyphénoloxydase naturelle, donne une coloration jaune orangée et la dihydroxyphénylanaline (L. DOPA) donne de la mélanine. L'avantage principal de ces catalyseurs enzymatiques consiste en l'obtention de pigments de couleurs et de nuances originales, sans utilisation de composés oxydants. Cependant, l'inconvénient majeur de ce procédé de coloration est l'utilisation d'enzymes, pour lesquels les craintes sur l'innocuité, la stabilité dans les compositions, la reproductibilité, le prix et l'immobilisation souvent nécessaire sont des facteurs qui limitent grandement leurs applications.

Ces compositions donnent des résultats qui ne sont pas totalement satisfaisants au niveau de la tenue et de la couleur.

Il est donc souhaitable de disposer de compositions tinctoriales qui, en coloration capillaire, donnent d'excellents résultats, tant du point de vue de la couleur que de celui de la tenue, et pour lesquelles on peut s'affranchir, pour la révélation de la coloration, de l'emploi d'agents d'oxydation classiques, en particulier du peroxyde d'hydrogène ou de l'emploi de systèmes enzymatiques.

La demanderesse a trouvé, de manière tout à fait surprenante, qu'il était possible d'atteindre ce but avec une composition qui comprend un précurseur de colorant choisi parmi les composés comportant au moins un cycle aromatique ayant au moins deux groupes hydroxyles portés par deux atomes de carbone consécutifs du cycle aromatique, une base d'oxydation de type para ou ortho choisi parmi les amines aromatiques, et un système catalytique chimique comprenant un premier constituant choisi parmi les sels et oxydes de Mn(II) et/ou de Zn(II) et leurs mélanges, et un second constituant choisi parmi les hydrogénocarbonates alcalins, les hydrogénocarbonates alcalino-terreux et leurs mélanges.

Ainsi, le système catalytique chimique présent dans la composition de l'invention se comporte comme une pseudo-oxydase capable de mimer l'activité oxydase sans les inconvénients liés à l'emploi d'un système enzymatique.

En particulier, cette composition de coloration, destinée notamment à la coloration des fibres kératiniques, ne nécessite pas la présence d'enzymes, ni de peroxyde d'hydrogène.

La présente invention concerne également un procédé pour révéler la coloration d'une composition de base peu ou pas colorée comprenant au moins un précurseur de colorant par oxydation, et au moins une base d'oxydation pour nuancer la coloration obtenue, qui consiste à ajouter à la composition de base un système catalytique purement chimique et à mettre la composition de base additionnée du système catalytique en présence d'un milieu contenant ou générant de l'oxygène.

La présente invention concerne en outre un procédé de coloration des fibres kératiniques utilisant une composition telle que définie ci-dessus.

Enfin, la présente invention concerne des conditionnements et des formes galéniques de la composition de coloration ou de constituants de la composition de coloration selon l'invention

La composition pour la coloration de la peau et/ou des fibres kératiniques selon l'invention comprend, dans un milieu physiologiquement acceptable, une quantité efficace d'au moins un précurseur de colorant choisi parmi les composés comportant au moins un cycle aromatique ayant au moins deux groupes hydroxyles (OH) portés par deux atomes de carbone consécutifs du cycle aromatique, une quantité efficace d'au moins une base d'oxydation de type para ou ortho, choisie parmi les amines aromatiques, et une quantité efficace d'un système catalytique comprenant un premier constituant choisi parmi les sels et oxydes de Mn(II), et/ou Zn(II) et leurs mélanges et un second constituant choisi parmi les hydrogénocarbonates alcalins, les hydrogénocarbonates alcalino-terreux et leurs mélanges, les proportions du premier constituant et du second constituant étant telles que:

$$\frac{[Mn(II)]}{[HCO_3]} \leq 1 \text{ avec } [\text{Mn(II)}] \neq 0$$

$$\frac{[Zn(II)]}{[HC0_3]} \leq 1 \text{ avec } [\text{Zn(II)}] \neq 0$$

$$\frac{[Mn(II) + Zn(II)]}{[HCO_3]} \leq 1 \text{ avec } [\text{Mn(II)}] \text{ et } [\text{Zn(II)}] \neq 0$$

où [Mn(II)], [Zn(II)] et [$HCO_3$] représentent respectivement les concentrations molaires en Mn(II), Zn(II) et $HCO_3$ dans la composition.

Généralement, le rapport $\frac{[Mn(II)]}{[HCO_3]}$ varie de $10^{-5}$ à $10^{-1}$, de préférence de $10^{-3}$ à $10^{-2}$ et est typiquement de l'ordre de $5.10^{-3}$.

Dans le cas où on utilise un sel ou un oxyde de Zn(II) seul, le rapport $\frac{[Zn(II)]}{[HCO_3]}$ est en général d'un ordre de 10 à 100 fois supérieur au rapport dans le cas de l'emploi d'un sel ou oxyde de Mn(II) seul.

Typiquement, ce rapport est de $10^{-4}$ ou plus, de préférence $10^{-3}$ ou plus, et de préférence de l'ordre de $5.10^{-1}$.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Généralement, la concentration molaire en Mn(II), Zn(II) ou Mn(II)+Zn(II) dans la composition finale varie de $10^{-3}$ à 10 mM/l, de préférence de $10^{-2}$ à 1 mM/l.

Lorsqu'on utilise seulement un ou plusieurs sels ou oxydes de Mn(II), la concentration molaire en Mn(II) dans la composition finale est typiquement de $10^{-3}$ à $10^{-1}$ mM/l, et de préférence de $10^{-2}$ à $10^{-1}$ mM/l.

De préférence, lorsque le système catalytique comporte seulement un ou plusieurs sels ou oxydes de Zn(II), la concentration en Zn(II) dans la composition finale est de $5.10^{-2}$ à 10 mM/l, mieux de $5.10^{-1}$ à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétrahydraté, le lactate de Mn(II) trihydraté, le phosphate de Mn(II), l'iodure de Mn(II), le nitrate de Mn(II) trihydraté, le bromure de Mn(II) et le perchlorate de Mn(II) tétrahydraté, et le sulfate de Mn(II) monohydraté.

Les sels particulièrement préférés sont $MnCl_2$ et $ZnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na.

Comme indiqué précédemment, le système catalytique chimique selon l'invention constitue une pseudo-oxydase spécifique qui permet l'oxydation des orthodiphénols, en présence d'oxygène, comme le feraient certains catalyseurs enzymatiques naturels ayant une activité polyphénoloxydase.

Par contre, le système catalytique selon l'invention n'a pas d'activité pseudocatalase en ce sens qu'il ne provoque pas la dismutation du peroxyde d'hydrogène à 0,3% en poids (soit 1 volume d'oxygène).

Les précurseurs de colorant des compositions de l'invention sont des composés ou mélanges de composés comprenant au moins un cycle aromatique, de préférence un cycle benzénique, comportant au moins deux groupes hydroxyles (OH) portés par deux atomes de carbone consécutifs du cycle aromatique.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Préférentiellement, les précurseurs de colorant selon l'invention peuvent être représentés par la formule (I) :

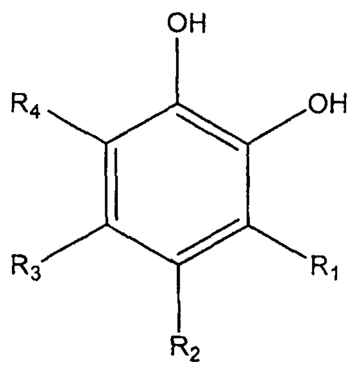

(I)

dans laquelle les substituants $R_1$ à $R_4$, identiques ou différents; représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical hétérocyclique éventuellement substitué, un radical contenant un ou plusieurs atomes de silicium, où deux des substituants $R_1$ à $R_4$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_1$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20,}$ tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_2$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphénylsiloxane, polydiméthylphénylsiloxane, stéaroxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocycliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les précurseurs de colorant préférés sont :

- les flavanols comme la catéchine et le gallate d'épichatéchine,
- les flavonols comme la quercétine,
- les anthocyanidines comme la péonidine,
- les anthocyanines, par exemple l'oenine,
- les hydroxybenzoates, par exemple les sels d'acide gallique,
- les flavones comme la lutéoline,

- les iridoïles comme l'oleuropéine,
  ces produits pouvant être osylés (par exemple glucosylés) et/ou sous forme d'oligomères (procyanidines),
- les hydroxystilbènes, par exemple le tétrahydroxy-3,3',4,5'-stilbène, éventuellement oxylés (par exemple glucosylés),
- la 3,4-dihydroxyphénylalanine et ses dérivés,
- la 2,3-dihydroxyphénylalanine et ses dérivés,
- la 4,5-dihydroxyphénylalanine et ses dérivés,
- le 4,5-dihydroxyindole et ses dérivés,
- le 5,6-dihydroxyindole et ses dérivés,
- le 6,7-dihydroxyindole et ses dérivés,
- le 2,3-dihydroxyindole et ses dérivés,
- les dihydroxycinnamates tels que l'acide caféique et l'acide chlorogénique,
- les hydroxycoumarines,
- les hydroxyisocoumarines,
- les hydroxycoumarones,
- les hydroxyisocoumarones,
- les hydroxychalcones,
- les hydroxychromones,
- les anthocyanes,
- les quinones,
- les hydroxyxanthones,
- les 1,2 dihydroxybenzènes,
- les 1,2,4 trihydroxybenzènes,
- les 1,2,3 trihydroxybenzènes,
- le 2,4,5 trihydroxytoluène,
- la 5,6 dihydroxyindoline, et
- les mélanges de ceux-ci.

Lorsque les précurseurs de colorant présentent des formes D et L, les deux formes peuvent être utilisées dans les compositions selon l'invention, ainsi que les racémiques.

La quantité de précurseurs de colorant dans la composition finale doit être suffisante pour obtenir une coloration visible. Cette quantité peut varier dans de larges mesures en fonction de la nature du précurseur et de l'intensité voulue pour la coloration.

En général, on obtient une coloration convenable lorsque le précurseur de colorant représente au moins 0,001% en poids du poids total de la composition.

En faisant varier la nature des différents précurseurs de colorant et leurs proportions dans la composition, on peut faire varier la couleur de la composition de coloration finale. On obtient ainsi une palette de couleurs.

Par exemple, avec un ratio 1/10 d'acide chlorogénique et de catéchine, on obtient une coloration marron claire et avec un ratio 1/1 une coloration acajou.

Les polymères formés en particulier avec la catéchine, l'acide gallique et leurs dérivés (tannins) ont des propriétés antimicrobiennes par emprisonnement des microorganismes lors de la polymérisation. Ces tannins ont également des propriétés astringentes intéressantes pour la peau.

Les précurseurs de colorants peuvent être des extraits de plantes, fruits, agrumes, légumes et des mélanges de ces extraits, qui contiennent de nombreux polyphénols tels que définis précédemment.

Parmi les extraits de plantes, on peut citer les extraits de rose et de thé.

Parmi les extraits de fruits, on peut citer les extraits de pomme, de raisin (en particulier de pépins de raisin) et de banane.

Parmi les extraits de légumes, on peut citer l'extrait de pomme de terre.

On peut également utiliser des mélanges d'extraits de plantes et/ou de fruits tels que des mélanges d'extraits de pomme et de thé et des mélanges d'extraits de raisin et de pomme.

Suivant les parties de fruits utilisés, par exemple pulpe ou pépins de raisin, la coloration obtenue est différente.

Les bases d'oxydation, de type para ou ortho, sont des composés qui ne sont pas des colorants en eux-mêmes, mais forment un colorant par un processus de condensation oxydative, soit sur eux-mêmes, soit en présence d'un coupleur ou modificateur. Ils comportent des groupements fonctionnels, soit deux groupements amino, soit un groupement amino et un groupement hydroxy en position para ou ortho l'un par rapport à l'autre.

La nature de ces bases d'oxydation n'est pas critique. Elles peuvent notamment être choisies parmi les ortho- et para-phénylènediamines, les bases doubles, les ortho- et para-aminophénols, les bases hétérocycliques ainsi que les sels d'addition de tous ces composés avec un acide.

A titre de paraphénylènediamines, on peut notamment citer les paraphénylènediamines de formule (II) suivante et leurs sels d'addition avec un acide :

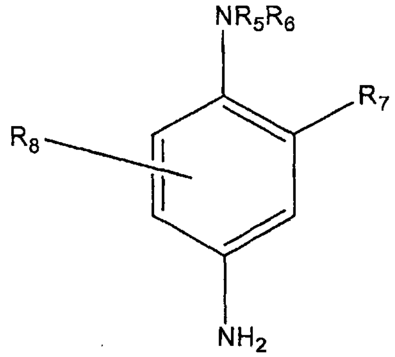

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;,
- $R_8$ représente un atome d'hydrogène, d'halogène ou un radical alkyle en $C_1$-$C_4$;
- $R_5$ et $R_6$ peuvent également former avec l'atome d'azote qui les porte un hétérocycle azoté à 5 ou 6 chaînons éventuellement substitué par un ou plusieurs groupements alkyle, hydroxy ou uréido.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (II) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl) amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide :

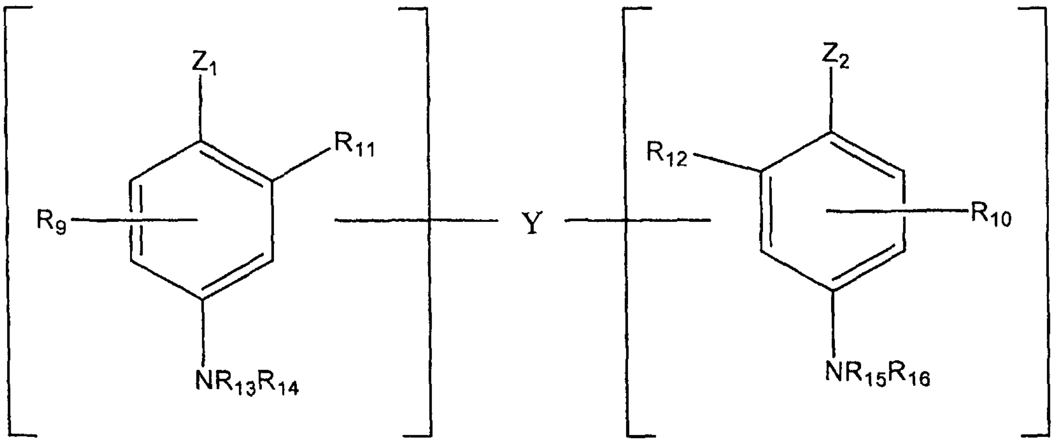

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ; étant entendu que les composés de formule (III) ne comportent qu'un seul bras de liaison Y par molécule.

Parmi les groupements azotés de la formule (III) ci-dessus, on peut citer notamment les radicaux amino, monoalkyl($C_1$-$C_4$)amino, dialkyl($C_{1-}C_4$)amino, trialkyl($C_1$-$C_4$)amino, monohydroxyalkyl($C_1$-$C_4$)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols, on peut notamment citer les para-aminophénols répondant à la formule (IV) suivante, et leurs sels d'addition avec un acide :

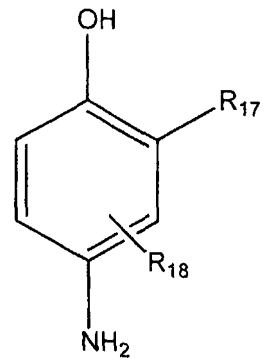

(IV)

dans laquelle :

- $R_{17}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_{1-}C_4$) ou aminoalkyle en $C_1$-$C_4$, ou hydroxyalkyl($C_1$-$C_4$)aminoalkyle en $C_1$-$C_4$, et
- $R_{18}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$) alkyle($C_{1-}C_4$).

Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB-1026978 et GB-1153196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE-2359399 ou japonais JP 88-169571 et JP-9110659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5,N7,N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE-3843892, DE-4133957 et demandes de brevet WO 94/08969, WO 94/08970, FR-A-2 733 749 et DE-19543988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le

4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Le milieu physiologiquement acceptable est un milieu solide ou liquide ne nuisant pas à la propriété de coloration des précurseurs ni à l'effet catalytique du système catalytique.

Le milieu physiologiquement acceptable est de préférence un milieu solubilisant des précurseurs de colorant.

Parmi les solvants des précurseurs convenant pour la formulation des compositions selon l'invention, on peut citer l'eau, les alcools, les éthers de polyols et leurs mélanges.

Parmi ces solvants, on peut citer à titre d'exemple, les alcanols inférieurs en $C_1$-$C_4$, tels que l'éthanol et l'isopropanol; les polyols ou éthers de glycols, comme le 2 butoxyéthanol, l'éthylèneglycol, la glycérine, le propylèneglycol, le monoéthyléther et le monométhyléther de diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les solvants sont de préférence des alcanols inférieurs ($C_1$-$C_6$) tels que l'éthanol et l'isopropanol et les alcanediols tels que le propylène glycol, la glycérine et le pentane diol.

Le milieu physiologiquement acceptable comprend de préférence de l'eau (en particulier distillée ou permutée) ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20% en poids.

La composition tinctoriale prête à l'emploi conforme à l'invention peut renfermer un ou plusieurs coupleurs choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide, ces composés étant différents des composés orthodihydroxylés de l'invention.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale prête à l'emploi et encore plus préférentiellement de 0,001 à 10% environ.

Les bases d'oxydation et les coupleurs constituent des colorants d'oxydation. Les sels d'addition avec un acide de ces colorants d'oxydation sont notamment choisis parmi les chlorhydrates, les bromhydrates, les sulfates et les tartrates, les lactates et les acétates.

Selon une forme de réalisation préférée, la composition tinctoriale conforme à l'invention peut en outre renfermer un ou plusieurs colorants directs notamment pour modifier les nuances en les enrichissant de reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition tinctoriale peuvent en outre plus particulièrement contenir, au moins un agent tensio-actif dans la proportion d'au moins 0,01 % en poids et de préférence un agent tensio-actif de nature non-ionique.

Les agents tensio-actifs peuvent être choisis parmi les agents tensio-actifs anioniques, cationiques, non-ioniques, amphotères ou leurs mélanges, et de préférence parmi les tensio-actifs non ioniques.

Parmi ces agents tensio-actifs, on peut citer les alkylbenzènesulfonates, les alkylnaphtalènesulfonates, les sulfates, les éthers sulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les quantités d'agents tensio-actifs présents dans la composition selon l'invention peuvent varier de 0,01 à

40% et de préférence de 0,5 à 30% du poids total de la composition.

Les agents épaississants que l'on peut ajouter dans les compositions conformes à l'invention, peuvent être choisis parmi l'alginate de sodium, la gomme arabique, les dérivés de cellulose, les polymères d'acide acrylique, la gomme de xanthane. On peut également utiliser les agents épaississants minéraux tels que la bentonite.

Ces agents épaississants sont présents de préférence dans des proportions comprises entre 0,1 et 5% et en particulier entre 0,2 et 3% en poids par rapport au poids total de la composition.

Ces compositions peuvent également contenir d'autres adjuvants cosmétiquement acceptables, tels que par exemple des agents de pénétration, des parfums, des tampons, etc.

Les compositions selon l'invention peuvent également comprendre tout adjuvant classique, en proportion usuelle, qui ne nuit pas aux propriétés recherchées, en particulier à l'effet colorant des compositions.

La composition colorante peut encore contenir une quantité efficace d'autres agents, par ailleurs antérieurement connus en coloration d'oxydation, tels que divers adjuvants usuels comme des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ces filtres UV organiques peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4,367,390, EP-0.863.145, EP-0.517.104, EP-0.570.838, EP-0.796.851, EP-0.775.698, EP-0.878.469 , EP-0.933.376 et EP-0.893.119 ; les dérivés de la benzophénone ; les dérivés de β,β'-diphénylacrylate, les dérivés de benzimidazole ; les dérivés bis-benzoazolyle tels que ceux décrits dans les brevets EP-0.669.323 et US 2,463,264 ; les dérivés de méthylène bis-(hydroxyphénylbenzotriazole) tels que ceux décrits dans les demandes US 5,237,071, US 5,166,355, GB-2303549, DE-19726184 et EP-0.893.119 ; les dérivés de l'acide p-aminobenzoïque ; les dimères dérivés d'α-alkylstyrène tels que décrits dans la demande de brevet DE-19855649 ; les polymères hydrocarbonés filtres et les silicones filtres tels que ceux décrits notamment dans la demande WO 93/04665. On peut aussi utiliser des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-0.518.772 et EP-0.518.773.

Bien entendu, l'homme de l'art veillera à choisir le ou les éventuels composés complémentaires mentionnés précédemment, de manière telle que les propriétés avantageuses attachées intrinsèquement à la composition tinctoriale selon l'invention ne soient pas, ou substantiellement pas, altérées par la ou les adjonctions envisagées.

De préférence, les compositions selon l'invention sont exemptes d'agents de chélation des sels de Mn(II) et/ou Zn(II) utilisés, car ces agents tendent à inhiber l'oxydation des précurseurs de colorant.

Les compositions tinctoriales de l'invention ont de préférence un pH qui varie de 3 à 12, de préférence de 6 à 9, et typiquement de l'ordre de 8. Ce pH, proche de la neutralité, associé à l'absence d'emploi de peroxydes, permet d'utiliser ces compositions pour une coloration des cuirs chevelus sensibles. Ce pH est ajusté par l'utilisation d'agents alcalinisants ou acidifiants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

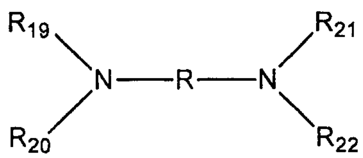
(V)

dans laquelle :

- R est un reste propylène éventuellement substitué par un groupement hydroxyle ou un radical alkyle en $C_1$-$C_4$ ; et
- $R_{19}$, $R_{20}$, $R_{21}$ et $R_{22}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide

chlorhydrique, l'acide orthophosphorique, des acides carboxyliques comme l'acide tartrique, l'acide citrique, l'acide lactique, ou des acides sulfoniques.

Pour révéler la coloration des compositions suivant l'invention, il suffit de mettre la composition contenant au moins un précurseur de colorant et une base d'oxydation et une quantité efficace du système catalytique selon l'invention, en présence d'un milieu oxydant tel qu'un milieu contenant de l'oxygène (par exemple l'oxygène de l'air).

Les compositions selon l'invention sont utiles pour la coloration des fibres kératiniques tels que les cheveux, les cils, les sourcils et les poils.

Pour la coloration des fibres kératiniques, différents procédés d'application des compositions selon l'invention peuvent être utilisés.

Selon un premier procédé, on applique sur les fibres kératiniques, en présence d'oxygène, par exemple l'oxygène de l'air, une composition comprenant tous les ingrédients de la composition de l'invention.

Selon un deuxième procédé, on peut en premier lieu appliquer sur les fibres kératiniques une première composition d'un ou plusieurs précurseurs de colorant dans un milieu physiologiquement acceptable, puis sur cette première composition, une deuxième composition contenant le système catalytique dans un milieu physiologiquement acceptable, qui en présence d'oxygène, révélera la coloration, la ou les bases d'oxydation étant contenues dans l'une ou l'autre des compositions.

On peut bien évidemment inverser l'ordre d'application des compositions.

L'application des compositions peut se faire par tout moyen connu, en particulier par pulvérisation.

La coloration de la composition peut être déterminée par le choix des précurseurs de colorant.

Les compositions selon l'invention peuvent se présenter et être conditionnées sous différentes formes.

Selon une première réalisation, les compositions selon l'invention peuvent être conditionnées sous forme d'aérosol à un seul compartiment dans lequel se trouvent la composition renfermant le ou les précurseurs de colorant, la ou les bases d'oxydation, le système catalytique et un gaz propulseur inerte classique tel que de l'azote, un hydrocarbure saturé comme l'isopropane ou un hydrocarbure halogéné.

Dans une seconde réalisation, la composition selon l'invention peut être conditionnée sous forme d'un kit comportant deux conteneurs distincts, l'un pour la composition de base contenant le ou les précurseurs de colorant, et la ou les bases d'oxydation, l'autre pour le système catalytique, la composition de base et le système catalytique étant mélangés ou appliqués successivement au moment de l'emploi.

Dans une troisième réalisation, la composition selon l'invention peut être également conditionnée sous forme d'un kit comportant deux conteneurs distincts, l'un pour la composition de base contenant le ou les précurseurs de colorant, l'autre pour le système catalytique et la ou les bases d'oxydation, la composition de base et le système catalytique étant mélangés ou appliqués successivement au moment de l'emploi.

Dans une quatrième réalisation, la composition peut être contenue dans un système à pompe à un seul compartiment, sans reprise d'air, ou dans un système à pompe à deux compartiments, le précurseur de colorant étant dans un compartiment et le système catalytique dans l'autre, la ou les bases d'oxydation étant contenues dans l'un ou l'autre de ces compartiments.

Dans une cinquième réalisation, la composition selon l'invention peut se présenter sous forme d'un ou plusieurs sachets imperméables à l'oxygène. Ce ou ces sachets seront, de manière avantageuse, des sachets en aluminium.

Si la composition selon l'invention se présente sous forme d'un sachet unique, ce dernier contiendra le ou les précurseurs de colorant, la ou les bases d'oxydation et le système catalytique.

Si la composition selon l'invention se présente sous forme de deux sachets, le premier sachet contiendra le système catalytique et le deuxième sachet contiendra le ou les précurseurs de colorant, la ou les bases d'oxydation étant contenus soit dans le premier sachet, soit dans le deuxième sachet.

## EXEMPLE:

On a réalisé des essais de coloration de mèches de cheveu. Pour cela, on a préparé plusieurs formulations de composition de coloration selon l'invention. Les formulations sont données dans le tableau 1 ci-après.

La diaminopyrazole correspond à la formule (VI) :

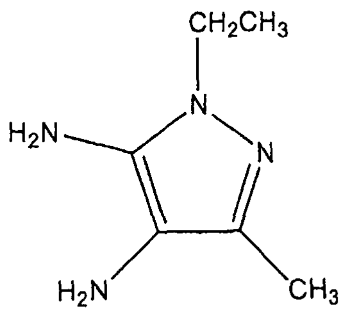

(VI)

| Formulation N° | Premier précurseur de colorant | | Base d'oxydation | | Système catalytique [$MnCl_2 + NaHCO_3$] | | |
|---|---|---|---|---|---|---|---|
| | Nature | Quantité | Nature | Quantité | Concentration $MnCl_2$ | Concentration $NaHCO_3$ | Quantité |
| 1 | catéchine | 25 mg | paraphénylène-diamine | 25 mg | 10 mM/l | 1M | 10 ml |
| 2 | catéchine | 25 mg | paraaminophénol | 25 mg | 10 mM/l | 1M | 10 ml |
| 3 | catéchine | 25 mg | diaminopyrazole | 25 mg | 10 mM/l | 1M | 10 ml |

Chacune des formulations 1 à 3 a été appliquée sur une mèche de cheveu de couleur gris naturel. On a laissé incuber les mèches pendant une heure puis on les a rincées à l'eau chaude du robinet.

On obtient avec les formulations 1 à 3, les résultats suivants récapitulés dans le tableau 2:

TABLEAU 2

| Formulation n° | Couleur |
|---|---|
| 1 | brun-roux |
| 2 | chatain clair |
| 3 | chatain foncé-noir |

L'association d'orthodiphénols avec une base d'oxydation donne de nouvelles nuances de couleur.

Enfin, un pH proche de la neutralité des compositions associé à l'absence de peroxydes permet d'envisager une utilisation pour la coloration des cheveux de personnes ayant des cuirs chevelus sensibles.

## Revendications

1. Composition pour la coloration des fibres kératiniques, **caractérisée en ce qu'**elle comprend, dans un milieu physiologiquement acceptable, une quantité efficace d'au moins un précurseur de colorant choisi parmi les composés comportant au moins un cycle aromatique ayant au moins deux groupes hydroxyles portés par deux atomes de carbone consécutifs du cycle aromatique, une quantité efficace d'au moins une base d'oxydation, de type para ou ortho choisi parmi les amines aromatiques, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituant étant telles que:

$$\frac{[Mn(II)]}{[HCO_3]} \leq 1 \text{ avec } [\mathrm{Mn(II)}] \neq 0$$

$$\frac{[Zn(II)]}{[HC0_3]} \leq 1 \text{ avec } [\mathrm{Zn(II)}] \neq 0$$

$$\frac{[Mn(II) + Zn(II)]}{[HCO_3]} \leq 1 \text{ avec } [\mathrm{Mn(II)}] \text{ et } [\mathrm{Zn(II)}] \neq 0$$

où [Mn(II)], [Zn(II)] et [$HCO_3$] représentent respectivement les concentrations molaires en Mn(II), Zn(II) et $HCO_3$ dans la composition.

2. Composition selon la revendication 1, **caractérisée en ce que** le rapport $\frac{[Mn(II)]}{[HCO_3]}$ varie de $10^{-5}$ à $10^{-1}$, de préférence de $10^{-3}$ à $10^{-2}$ et mieux est de l'ordre de $5.10^{-3}$.

3. Composition selon la revendication 1 ou 2, **caractérisée en ce que** le rapport $\frac{[Zn(II)]}{[HCO_3]}$ varie de $10^{-4}$ à < 1, de préférence de $10^{-3}$ à < 1, et mieux est de l'ordre de $5.10^{-1}$.

4. Composition selon l'une quelconque des revendications précédentes, **caractérisée en ce que** le rapport $\frac{[Mn(II) + Zn(II)]}{[HCO_3]}$ varie de $10^{-5}$ à $10^{-1}$, de préférence $10^{-3}$ à $10^{-2}$.

5. Composition selon l'une quelconque des revendications précédentes, **caractérisée en ce que** les sels de Mn(II)

et de Zn(II) sont choisis parmi les chlorure, fluorure, iodure, sulfate, phosphate, nitrate, perchlorate, les sels d'acides carboxyliques et leurs mélanges.

**6.** Composition selon la revendication 5, **caractérisée en ce que** le sel de Mn(II) et/ou de Zn(II) est le chlorure.

**7.** Composition selon la revendication 5, **caractérisée en ce que** les sels d'acides carboxyliques sont des sels d'acides carboxyliques hydroxylés.

**8.** Composition selon la revendication 7, **caractérisée en ce que** le sel d'acide carboxylique hydroxylé est le gluconate.

**9.** Composition selon l'une quelconque des revendications précédentes, **caractérisée en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

**10.** Composition selon l'une quelconque des revendications précédentes, **caractérisée en ce que** le cycle aromatique comportant au moins deux groupes hydroxyles sur deux atomes de carbone consécutifs du premier précurseur de colorant est un cycle benzénique ou un cycle aromatique condensé.

**11.** Composition selon la revendication 10, **caractérisée en ce que** le précurseur de colorant est un composé de formule (I) :

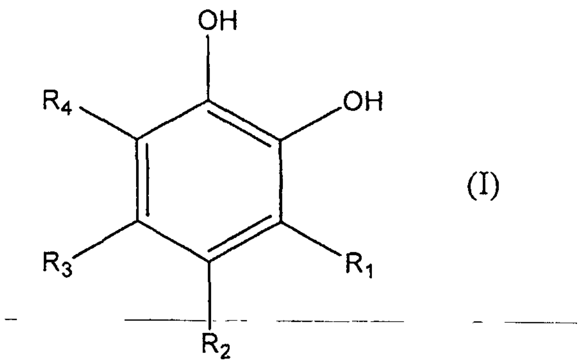

(I)

dans laquelle les substituts $R_1$ à $R_4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant éventuellement un ou plusieurs atomes de silicium, ou deux des substituants $R_1$ à $R_4$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

**12.** Composition selon l'une quelconque des revendications 1 à 10, **caractérisée en ce que** le précurseur de colorant est choisi parmi les flavanols, les flavonols, les anthocyaninidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4, 5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, les 1,2-dihydroxybenzènes, les 1,2,4-trihydroxybenzènes, les 1,2,3-trihydroxybenzènes, le 2,4,5-trihydroxytoluène, le 5,6-dihydroxyindoline et les mélanges de deux ou plus des composés précédents.

**13.** Composition selon l'une quelconque des revendications 1 à 10, **caractérisée en ce que** le précurseur de colorant est choisi parmi les extraits de plantes, de fruits, d'agrumes, de légumes et leurs mélanges.

**14.** Composition selon la revendication 13, **caractérisée en ce que** le précurseur de colorant est choisi parmi les extraits de thé, de raisin, de pomme, de banane, de pomme de terre et leurs mélanges.

**15.** Composition selon l'une quelconque des revendications précédentes, **caractérisée en ce que** le précurseur de colorant est présent à raison d'au moins 0,001 % du poids total de la composition.

**16.** Composition selon l'une quelconque des revendications précédentes, **caractérisée en ce que** la base d'oxydation peut être choisie parmi les ortho- et para-phénylènediamines, les bases doubles, les ortho- et para-aminophénols, les bases hétérocycliques, ainsi que les sels d'addition de tous ces composés avec un acide.

**17.** Composition selon la revendication 16, **caractérisée en ce que** la base d'oxydation est choisie parmi les para-phénylènediamines répondant à la formule (II):

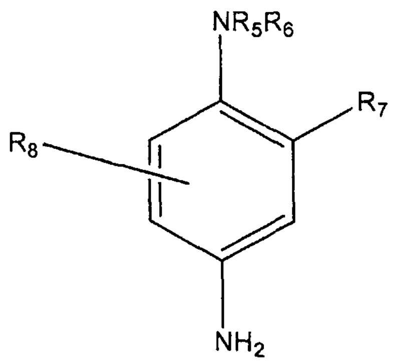

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;
- $R_8$ représente un atome d'hydrogène, d'halogène ou un radical alkyle en $C_1$-$C_4$ ;
- $R_5$ et $R_6$ peuvent également former avec l'atome d'azote qui les porte un hétérocycle azoté à 5 ou 6 chaînons éventuellement substitué par un ou plusieurs groupements alkyle, hydroxy ou uréido.

**18.** Composition selon la revendication 17, **caractérisée en ce que** les paraphénylènes diamines sont choisies parmi la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

**19.** Composition selon la revendication 16, **caractérisée en ce que** les bases doubles sont choisies parmi les composés répondant à la formule (III) :

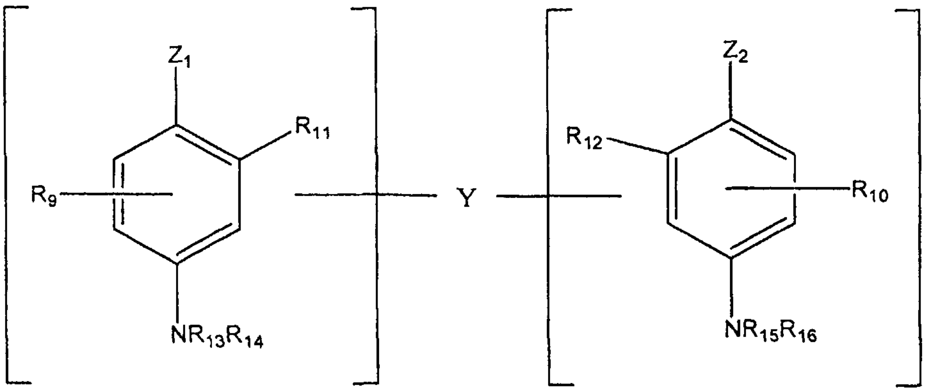

(III)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ;

étant entendu que les composés de formule (III) ne comportent qu'un seul bras de liaison Y par molécule.

**20.** Composition selon la revendication 19, **caractérisée en ce que** les bases doubles sont choisies parmi le N, N'-bis-(β-hydroxyéthyl) N,N'-bis-(4'-aminophényl) 1,3-diamino propanol, la N,N'-bis-(β-hydroxyéthyl) N, N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, 1 la N, N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

**21.** Composition selon la revendication 16, **caractérisée en ce que** la base d'oxydation est choisie parmi les para-aminophénols répondant à la formule (IV) :

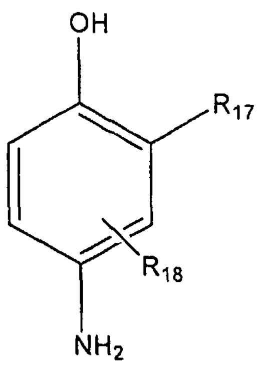

(IV)

dans laquelle :

- $R_{17}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)ami-

noalkyle en $C_1$-$C_4$ ;

• $R_{18}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

**22.** Composition selon la revendication 21, **caractérisée en ce que** les para-aminophénols sont choisis parmi le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

**23.** Composition selon la revendication 16, **caractérisée en ce que** la base d'oxydation est un orthoaminophénol choisi parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

**24.** Composition selon la revendication 16, **caractérisée en ce que** la base d'oxydation est une base hétérocyclique choisie parmi les dérivés pyridiniques, les dérivés pyrimidiniques, les dérivés pyrazoliques, et leurs sels d'addition avec un acide.

**25.** Composition selon la revendication 24, **caractérisée en ce que** les dérivés pyridiniques sont choisis parmi la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, et la 3,4-diamino-pyridine.

**26.** Composition selon la revendication 24, **caractérisée en ce que** les dérivés pyrimidiniques sont choisis parmi la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino pyrazolo-[1,5-a]-pyrimidine; et leurs sels d'addition et leurs formes tautomères, lorsqu'il existe un équilibre tautomérique.

**27.** Composition selon la revendication 24, **caractérisée en ce que** les dérivés pyrazoliques sont choisis parmi le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)- pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le .4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, et le 3,5-diamino-4-(β-hydroxyéthyl)amino-1-méthyl-pyrazole.

**28.** Composition selon l'une quelconque des revendications précédentes, **caractérisée en ce que** la base d'oxydation représente de 0,0005 à 12% en poids du poids total de la composition, et de préférence de 0,005 à 8%.

**29.** Composition selon l'une quelconque des revendications précédentes, **caractérisée en ce que** le milieu physiologiquement acceptable est un milieu solubilisant, de préférence à propriété bactériostatique.

**30.** Composition selon l'une quelconque des revendications précédentes, **caractérisée en ce que** le milieu physiologiquement acceptable comprend un solvant ou un mélange de solvants.

**31.** Composition selon la revendication 30, **caractérisée en ce que** le solvant est choisi parmi l'eau, les alcools, les polyols, les éthers de polyols et leurs mélanges.

**32.** Composition selon la revendication 31, **caractérisée en ce que** l'alcool est un alcanol ou un alcanediol.

**33.** Composition selon la revendication 31 ou 32, **caractérisée en ce que** le solvant est un mélange eau/alcool.

**34.** Composition selon la revendication 33, **caractérisée en ce que** l'alcool représente jusqu'à 80% en poids du mélange, de préférence 1 à 50% en poids et mieux de 5 à 20% en poids.

**35.** Composition selon l'une quelconque des revendications précédentes, **caractérisée en ce qu'**elle contient un ou plusieurs coupleurs choisis parmi les familles de composés suivantes : les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

**36.** Composition selon la revendication 35, **caractérisée en ce que** les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

**37.** Composition selon la revendication 35 ou 36, **caractérisée en ce que** le ou les coupleurs représentent de 0,0001 à 15% en poids du poids total de la composition, et de préférence de 0,001 à 10%.

**38.** Composition selon l'une quelconque des revendications précédentes, **caractérisée en ce qu'**elle est exempte de tout agent de chélation du sel de Mn(II) et/ou Zn(II).

**39.** Composition selon l'une quelconque des revendications précédentes, **caractérisée en ce qu'**elle est conditionnée sous forme d'un aérosol ou d'un système à pompe sans reprise d'air.

**40.** Composition selon l'une quelconque des revendications 1 à 38, **caractérisée en ce qu'**elle se présente sous la forme de deux composants séparés, un premier composant comprenant le système catalytique et la base d'oxydation, dissous dans un milieu physiologiquement acceptable, et un deuxième composant comprenant le précurseur de colorant dissous dans un milieu physiologiquement acceptable.

**41.** Composition selon l'une quelconque des revendications 1 à 38, **caractérisée en ce qu'**elle se présente sous la forme de deux composants séparés, un premier composant comprenant le système catalytique dissous dans un milieu physiologiquement acceptable, et un deuxième composant comprenant le précurseur de colorant et la base d'oxydation dissous dans un milieu physiologiquement acceptable.

**42.** Composition selon les revendications 40 ou 41, **caractérisée en ce qu'**elle est conditionnée dans un système à pompe à deux compartiments distincts, le ou les précurseurs de colorant étant dans un compartiment et le système catalytique dans l'autre compartiment, et la ou les bases d'oxydation étant dans l'un ou l'autre de ces deux compartiments.

**43.** Composition selon l'une quelconque des revendications 1 à 38, **caractérisée en ce qu'**elle est conditionnée sous forme d'un ou plusieurs sachets imperméables à l'oxygène.

**44.** Composition selon la revendication 43, **caractérisée en ce qu'**elle est conditionnée sous forme de deux sachets imperméables à l'oxygène :

- un premier sachet comprenant le système catalytique et la ou les bases d'oxydation et un deuxième sachet comprenant le précurseur de colorant, ou
- un premier sachet comprenant le système catalytique et un deuxième sachet comprenant le ou les précurseurs de colorants et la ou les bases d'oxydation.

**45.** Procédé de coloration des fibres kératiniques, **caractérisé en ce qu'**il consiste à appliquer sur la peau et/ou les fibres kératiniques une couche d'une composition selon l'une quelconque des revendications 1 à 44.

**46.** Procédé selon la revendication 45, **caractérisé en ce que** la coloration est obtenue en appliquant sur les fibres kératiniques une première composition de base contenant un ou plusieurs précurseurs de colorant dans un milieu physiologiquement acceptable, puis en appliquant une composition comprenant le système catalytique dans un milieu physiologiquement acceptable, et vice-versa, la ou les bases d'oxydation étant soit contenues dans la composition de base, soit associées au système catalytique.

**47.** Procédé selon les revendications 45 ou 46, **caractérisé en ce que** les compositions sont appliqués par pulvérisation.

## Claims

**1.** Composition for the coloration of keratin fibres, **characterized in that** it comprises, in a physiologically acceptable medium, an effective quantity of at least one colouring material precursor selected from the compounds containing at least one aromatic ring having at least two hydroxyl groups borne by two adjacent carbon atoms of the aromatic ring, an effective quantity of at least one oxidation base of the para or ortho type selected from the aromatic amines, and an effective quantity of a catalytic system comprising a first constituent selected from the salts and oxides of Mn(II) and/or Zn(II) and their mixtures, and a second constituent selected from the alkali hydrogen carbonates, the alkaline earth hydrogen carbonates and their mixtures, the proportions of the first constituent and the second constituent being such that:

$$\frac{[Mn(II)]}{[HCO_3]} \leq 1 \text{ with } [Mn(II)] \neq 0$$

$$\frac{[Zn(II)]}{[HCO_3]} \leq 1 \text{ with } [Zn(II)] \neq 0$$

$$\frac{[Mn(II) + [Zn(II)]}{[HCO_3]} \leq 1 \text{ with } [Mn(II) \text{ and } [Zn(II)] \neq 0$$

where [Mn(II)], [Zn(II)] and [$HCO_3$] respectively represent the molar concentrations of Mn(II), Zn(II) and $HCO_3$ in the composition.

**2.** Composition according to Claim 1, **characterized in that** the ratio $\frac{[Mn(II)]}{[HCO_3]}$ is from $10^{-5}$ to $10^{-1}$, and preferably from $10^{-3}$ to $10^{-2}$ and is, more preferably of $5 \times 10^{-3}$.

**3.** Composition according to Claim 1 or 2, **characterized in that** the ratio $\frac{[Zn(II)]}{[HCO3]}$ is from $10^{-4}$ to < 1, preferably $10^{-3}$ to < 1, and is more preferably, of the $5 \times 10^{-1}$.

**4.** Composition according to any one of the preceding Claims,

**characterized in that** the ratio $\frac{[Mn(II) + [Zn(II)]}{[HCO_3]}$ is from $10^{-5}$ to $10^{-1}$, and preferably from $10^{-3}$ to $10^{-2}$.

5. Composition according to any one of the preceding Claims, **characterized in that** the salts of Mn(II) and Zn(II) are selected from chloride, fluoride, iodide, sulfate, phosphate, nitrate, perchlorate, carboxylic acid salts and their mixtures.

6. Composition according to Claim 5, **characterized in that** the salt of Mn(II) and/or Zn(II) is the chloride.

7. Composition according to Claim 5, **characterized in that** the salts of carboxylic acids are salts of hydroxylated carboxylic acids.

8. Composition according to Claim 7, **characterized in that** the hydroxylated carboxylic acid salt is gluconate.

9. Composition according to any one of the preceding Claims, **characterized in that** the hydrogen carbonate is selected from sodium hydrogen carbonate, potassium hydrogen carbonate, magnesium hydrogen carbonate, calcium hydrogen carbonate and their mixtures.

10. Composition according to any one of the preceding Claims, **characterized in that** the aromatic ring bearing at least two hydroxyl groups on two adjacent carbon atoms of the first colouring material precursor is a benzene ring or condensed aromatic ring.

11. Composition according to Claim 10, **characterized in that** the colouring material precursor is a compound of formula (I):

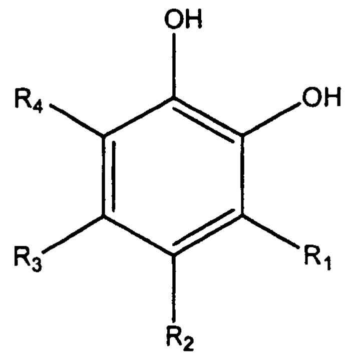

(I)

in which the substituents $R_1$ to $R_4$, identical or different, represent hydrogen, halogen, hydroxyl, carboxyl, alkyl carboxylate, optionally substituted amino, optionally substituted linear or branched alkyl, optionally substituted linear or branched alkenyl, optionally substituted cycloalkyl, alkoxy, alkoxyalkyl, alkoxyaryl, the aryl group being optionally substituted, aryl, substituted aryl, optionally substituted heterocyclic radical, a radical containing possibly one or more silicon atoms in which two of the substituents $R_1$ to $R_4$ form together a saturated or unsaturated ring optionally containing one or more heteroatoms and optionally condensed with one or more saturated or unsaturated rings optionally containing one or more heteroatoms.

12. Composition according to any one of Claims 1 to 10, **characterized in that** the colouring material precursor is selected from the flavanols, the flavonols, the anthocyanidines, the anthocyanines, the hydroxybenzoates, the flavones, the iridoids, these products being optionally glycosylated and/or in the form of oligomers, the hydroxystilbenes, optionally glycosylated, 3,4-dihydroxyphenylalanine and its derivatives, 2,3-dihydroxyphenylalanine and its derivatives, 4,5-dihydroxyphenylalanine and its derivatives, 4,5-dihydroxyindole and its derivatives, 5,6-dihydroxyindole and its derivatives, 6,7-dihydroxyindole and its derivatives, 2,3-dihydroxyindole and its derivatives, the dihydroxycinnamates, the hydroxycoumarins, the hydroxyisocoumarins, the hydroxycoumarones, the hydroxyisocoumarones, the hydroxychalcones, the hydroxychromones, the anthocyans, the quinones, the hydroxyxanthones, the 1,2-dihydroxybenzenes, the 1,2,4-trihydroxybenzenes, the 1,2,3-trihydroxybenzenes, the 2,4,5-trihydroxytoluene, the 5,6-dihydroxyindoline, and mixtures of two or more of the preceding compounds.

**13.** Composition according to any one of Claims 1 to 10, **characterized in that** the colouring material precursor is selected from extracts of plants, fruits, citrus fruits, vegetables and their mixtures.

**14.** Composition according to Claim 13, **characterized in that** the colouring material precursor is selected from the extracts of tea, grape, apple, banana, potato and their mixtures.

**15.** Composition according to any one of the preceding Claims, **characterized in that** the colouring material precursor is present to the extent of at least 0.001% of the total weight of the composition.

**16.** Composition according to any one of the preceding Claims, **characterized in that** the oxidation base may be selected from the ortho and para phenylenediamines, the double bases, the ortho and para aminophenols, the heterocyclic bases as well as the addition salts of all of these compounds with an acid.

**17.** Composition according to Claim 16, **characterized in that** the oxidation base is selected from the para-phenylenediamines corresponding to formula (II):

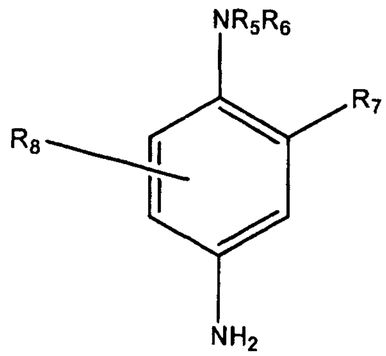

(II)

in which:

- $R_5$ represents hydrogen, $C_1$-$C_4$ alkyl, $C_1$-$C_4$ monohydroxyalkyl, $C_2$-$C_4$ polyhydroxyalkyl, $C_1$-$C_4$ alkoxy $C_1$-$C_4$ alkyl, $C_1$-$C_4$ alkyl, substituted by a nitrogen-containing group, phenyl or 4'-aminophenyl;
- $R_6$ represents hydrogen, $C_1$-$C_4$ alkyl, $C_1$-$C_4$ monohydroxyalkyl or $C_2$-$C_4$ polyhydroxyalkyl, $C_1$-$C_4$ alkoxy $C_1$-$C_4$ alkyl or $C_1$-$C_4$ alkyl, substituted by a nitrogen-containing group;
- $R_7$ represents hydrogen , halogen such as chlorine, a $C_1$-$C_4$ alkyl radical, sulfo, carboxy, $C_1$-$C_4$ monohydroxyalkyl or $C_1$-$C_4$ hydroxyalkoxy, $C_1$-$C_4$ acetylaminoalkoxy, $C_1$-$C_4$ mesylaminoalkoxy or $C_1$-$C_4$ carbamoylaminoalkoxy;
- $R_8$ represents hydrogen, halogen or $C_1$-$C_4$ alkyl;
- $R_5$ and $R_6$ may also form with the nitrogen atom which bears them a 5 or 6 membered nitrogen-containing heterocycle, optionally substituted by one or more alkyl, hydroxy or ureido groups.

**18.** Composition according to Claim 17, **characterized in that** the para-phenylenediamines are selected from para-phenylenediamine, para-toluylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl -para-phenylenediamine, N,N bis-(β-hydroxyethyl)-para-phenylenediamine 2-chloro-para-phenylenediamine, and their addition salts with an acid.

**19.** Composition according to Claim 16, **characterized in that** the double bases are selected from compounds corresponding to formula (III):

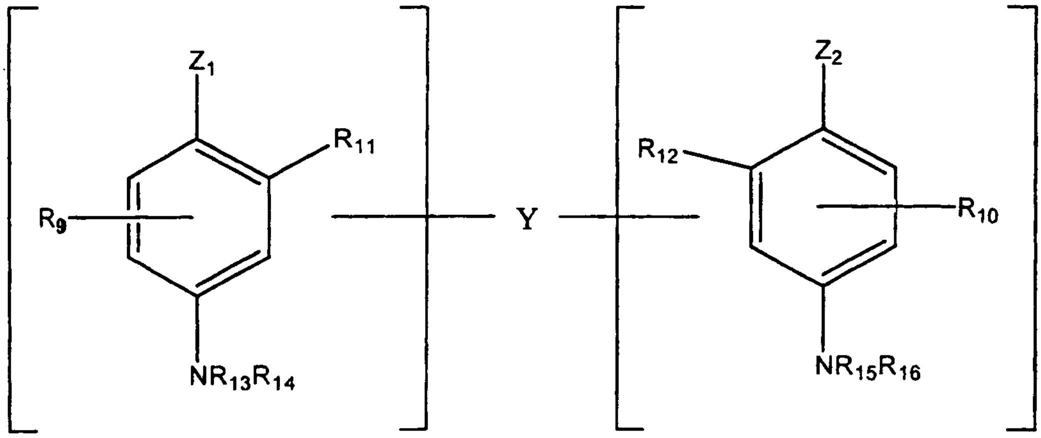

(III)

in which:

- $Z_1$ and $Z_2$, identical or different, represent a hydroxyl or $-NH_2$ radical which may be substituted by a $C_1$-$C_4$ alkyl radical or by a linking arm Y;
- the linking arm Y represents an alkylene chain comprising from 1 to 14 linear or branched carbon atoms which may be interrupted or terminated by one or more nitrogen-containing groups and/or by one or more heteroatoms such as oxygen, sulfur or nitrogen atoms, and optionally substituted by one or more hydroxyl or $C_1$-$C_6$ alkoxy radicals;
- $R_9$ and $R_{10}$ represent hydrogen or halogen, a $C_1$-$C_4$ alkyl radical, $C_1$-$C_4$ monohydroxyalkyl, $C_2$-$C_4$ polyhydroxyalkyl, $C_1$-$C_4$ aminoalkyl or a linking arm Y;
- $R_{11}$, $R_{12}$, $R_{13}$, $R_{14}$, $R_{15}$ and $R_{16}$, identical or different, represent hydrogen, a linking arm Y or a $C_1$-$C_4$ alkyl radical; it being understood that the compounds of formula (III) only bear a single linking arm Y per molecule.

**20.** Composition according to Claim 19, **characterized in that** the double bases are selected from N,N'-bis-(β-hydroxyethyl) N,N'-bis-(4'-aminophenyl) 1,3-diaminopropanol, N,N'-bis-(β-hydroxyethyl) N,N'-bis-(4'-aminophenyl) ethylenediamine, N,N'-bis-(4'-aminophenyl) tetramethylenediamine, N,N'-bis-(β-hydroxyethyl) N,N'-bis-(4'-aminophenyl) tetramethylenediamine, N,N'-bis-(4-methyl-aminophenyl) tetramethylenediamine, N,N'-bis-(ethyl) N, N'-bis-(4'-amino, 3'-methylphenyl) ethylenediamine, 1,8-bis-(2,5-diaminophenoxy)-3,5-dioxo-octane, and their addition salts with an acid.

**21.** Composition according to Claim 16, **characterized in that** the oxidation base is selected from the para-aminophenols corresponding to formula (IV):

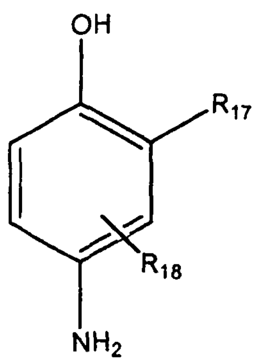

(IV)

in which:

- $R_{17}$ represents hydrogen, halogen such as fluorine, $C_1$-$C_4$ alkyl, $C_1$-$C_4$ monohydroxyalkyl, $C_1$-$C_4$ alkoxy $C_1$-$C_4$ alkyl or $C_1$-$C_4$ aminoalkyl, or $C_1$-$C_4$ hydroxyalkyl $C_1$-$C_4$ aminoalkyl, and

• $R_{18}$ represents hydrogen, halogen such as fluorine, $C_1$-$C_4$ alkyl, $C_1$-$C_4$ monohydroxyalkyl, $C_2$-$C_4$ polyhydroxyalkyl, $C_1$-$C_4$ aminoalkyl, $C_1$-$C_4$ cyanoalkyl or $C_{1-}C_4$ alkoxy $C_1$-$C_4$ alkyl.

**22.** Composition according to Claim 21, **characterized in that** the para-aminophenols are selected from para-aminophenol, 4-amino-3-methyl-phenol, 4-amino-3-fluoro-phenol, 4-amino-3-hydroxymethyl phenol, 4-amino-2-methyl phenol, 4-amino-2-hydroxymethyl phenol, 4-amino-2-methoxymethyl phenol, 4-amino-2-aminomethyl phenol, 4-amino-2-(β-hydroxyethyl-aminomethyl) phenol, and their addition salts with an acid.

**23.** Composition according to Claim 16, **characterized in that** the oxidation base is an ortho-aminophenol selected from 2-aminophenol, 2-amino-1-hydroxy-5-methyl-benzene, 2-amino-1-hydroxy-6-methyl-benzene, 5-acetamido-2-amino-phenol, and their addition salts with an acid.

**24.** Composition according to Claim 16, **characterized in that** the oxidation base is a heterocyclic base selected from pyridine derivatives, pyrimidine derivatives, pyrazole derivatives and their addition salts with an acid.

**25.** Composition according to Claim 24, **characterized in that** the pyridine derivatives are selected from 2,5-diamino pyridine, 2-(4-methoxyphenyl)amino-3-amino-pyridine, 2,3-diamino-6-methoxy pyridine, 2-(β-methoxyethyl) amino-3-amino-6-methoxy pyridine, and 3,4-diamino pyridine.

**26.** Composition according to Claim 24, **characterized in that** the pyrimidine derivatives are selected from 2,4,5,6-tetra-aminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine, pyrazolo-[1,5-a]-pyrimidine-3,7-diamine; 2,5-dimethyl-pyrazolo-[1,5-a]-pyrimidine-3,7-diamine, pyrazolo-[1,5-,a]-pyrimidine-3,5-diamine; 2,7-dimethyl-pyrazolo-[1,5-a]-pyrimidine-3,5-diamine; 3-amino-pyrazolo-[1,5-a]-pyrimidin-7-ol; 3-amino-pyrazolo-[1,5-a]-pyrimidin-5-ol; 2-(3-amino-pyrazolo-[1,5-a]-pyrimidin-7-ylamino)-ethanol; 2-(7-amino-pyrazolo-[1,5-a]-pyrimidin-3-ylamino)-ethanol; 2-[(3-amino-pyrazolo-[1,5-a]-pyrimidin-7-yl)-(2-hydroxy-ethyl-amino)-ethanol; 2-[(7-amino-pyrazolo-[1,5-a]-pyrimidin-3-yl)-(2-hydroxy-ethyl-amino)-ethanol; 5,6-dimethyl-pyrazolo-[1,5-a]-pyrimidine-3,7-diamine; 2,6-dimethyl-pyrazolo-[1,5-a]-pyrimidine-3,7-diamine; 2,5,N7,N7-tetramethyl- pyrazolo-[1,5-a]-pyrimidine-3,7-diamine; 3-amino-5-methyl-7-imidazolylpropylamino pyrazolo-[1,5-a]-pyrimidine; and their addition salts and their tautomeric forms, when a tautomeric equilibrium exists.

**27.** Composition according to Claim 24, **characterized in that** the pyrazole derivatives are selected from 4,5-diamino-1-methyl-pyrazole, 3,4-diamino-pyrazole, 4,5-diamino-1-(4'-chlorobenzyl)-pyrazole, 4,5-diamino-1,3-dimethylpyrazole, 4,5-diamino-3-rnethyl-1-phenyl pyrazole, 4,5-diamino-1-methyl-3-phenyl pyrazole, 4-amino-1,3-dimethyl-5-hydrazino-pyrazole, 1-benzyl-4,5-diamino-3-methyl pyrazole, 4,5-diamino-3-tert.butyl-1-methyl pyrazole, 4,5-diamino-1-tert.butyl-3-methyl pyrazole, 4,5-diamino-1-(β-hydroxyethyl)-3-methyl pyrazole , 4,5-diamino-1-(β-hydroxyethyl)- pyrazole, 4,5-diamino-1-ethyl-3-methyl pyrazole, 4,5-diamino-1-ethyl-3-(4'-methoxyphenyl)- pyrazole, 4,5-diamino-1-ethyl-3-hydroxymethyl pyrazole, 4,5-diamino-3-hydroxymethyl-1-methyl pyrazole, 4,5-diamino-3-hydroxymethyl-1-isopropyl pyrazole, 4,5-diamino-3-methyl-1-isopropyl pyrazole, 4-amino-5-(2'-aminoethyl)amino-1,3-dimethyl pyrazole, 3,4,5-triamino pyrazole, 1-methyl-3,4,5-triamino-pyrazole, 3,5-diamino-1-methyl-4-methylamino-pyrazole and 3,5-diamino-4-(β-hydroxyethyl)amino-1-methyl-pyrazole.

**28.** Composition according to any one of the preceding Claims, **characterized in that** the oxidation base represents from 0.0005 to 12% by weight of the total weight of the composition, and preferably from 0.005 to 8%.

**29.** Composition according to any one of the preceding Claims, **characterized in that** the physiologically acceptable medium is a solubilizing medium, preferably with bacteriostatic properties.

**30.** Composition according to any one of the preceding Claims, **characterized in that** the physiologically acceptable medium comprises a solvent or mixture of solvents.

**31.** Composition according to Claim 30, **characterized in that** the solvent is selected from water, the alcohols, the polyols, the polyol ethers and their mixtures.

**32.** Composition according to Claim 31, **characterized in that** the alcohol is an alkanol or an alkanediol.

**33.** Composition according to Claim 31 or 32, **characterized in that** the solvent is a water/alcohol mixture.

**34.** Composition according to Claim 33, **characterized in that** the alcohol represents up to 80% by weight of the mixture, and preferably 1 to 50% by weight and, more preferably, 5 to 20% by weight.

**35.** Composition according to any one of the preceding Claims, **characterized in that** it comprises one or more couplers selected from the following families of compounds: the meta-aminophenols, the meta-phenylenediamines, the meta-diphenols, the naphthols and the heterocyclic couplers such as for example indole derivatives, indoline derivatives, sesamol and its derivatives, pyridine derivatives, pyrazolotriazole derivatives, pyrazolones, indazoles, benzimidazoles, benzothiazoles, benzoxazoles, 1,3-benzodioxoles, quinolines and their addition salts with an acid.

**36.** Composition according to Claim 35, **characterized in that** the couplers are selected from 2,4-diamino-1-(β-hydroxyethyloxy) benzene, 2-methyl 5-amino phenol, 5-N-(β-hydroxyethyl) amino 2-methyl phenol, 3-amino phenol, 1,3-dihydroxybenzene, 1,3-dihydroxy 2-methyl benzene, 4-chloro 1,3-dihydroxybenzene, 2-amino 4-(β-hydroxyethylamino) 1-methoxybenzene, 1,3-diaminobenzene, 1,3-bis-(2,4-diaminophenoxy) propane, sesamol, 1-amino-2-methoxy-4,5-methylenedioxy benzene, α-naphthol, 6-hydroxyindole, 4-hydroxyindole, 4-hydroxy N-methyl indole, 6-hydroxyindoline, 2,6-dihydroxy 4-methyl pyridine, 1-H 3-methyl pyrazole 5-one, 1-phenyl 3-methyl pyrazol-5-one, 2-amino 3-hydroxypyridine, 3,6-dimethyl pyrazolo-[3,2-c]-1,2,4-triazole, 2,6-dimethyl-pyrazolo-[1,5-b]-1,2,4-triazole and their addition salts with an acid.

**37.** Composition according to Claim 35 or 36, **characterized in that** the coupler(s) represent(s) from 0.0001 to 15% by weight of the total weight of the composition, and preferably from 0.001 to 10%.

**38.** Composition according to any one of the preceding Claims, **characterized in that** it is free of any chelating agent for the Mn(II) and/or Zn(II) salt(s)

**39.** Composition according to any one of the preceding Claims, **characterized in that** it is packaged in the form of an aerosol or a pump system without intake of air.

**40.** Composition according to any one of Claims 1 to 38, **characterized in that** it is available in the form of two separate constituents, a first constituent comprising the catalytic system and the oxidation base, dissolved in a physiologically acceptable medium, and a second constituent comprising the colouring material precursor dissolved in a physiologically acceptable medium.

**41.** Composition according to any one of Claims 1 to 38, **characterized in that** it is available in the form of two separate constituents, a first constituent comprising the catalytic system dissolved in a physiologically acceptable medium, and a second constituent comprising the colouring material precursor and the oxidation base dissolved in a physiologically acceptable medium.

**42.** Composition according to Claims 40 or 41, **characterized in that** it is packaged in a pump system with two distinct compartments, the colouring material precursor(s) being in one compartment and the catalytic system being in the other compartment, and the oxidation base(s) being in one or other of these two compartments.

**43.** Composition according to any one of Claims 1 to 38, **characterized in that** it is packaged in the form of one or more bags impermeable to oxygen.

**44.** Composition according to Claim 43, **characterized in that** it is packaged in the form of two bags impermeable to oxygen:

- a first bag containing the catalytic system and the oxidation base(s) and a second bag containing the colouring material precursor, or
- a first bag containing the catalytic system and a second bag containing the colouring material precursor(s) and the oxidation base(s).

**45.** Method for the coloration of keratin fibres, **characterized in that** it consists of applying to the skin and/or keratin fibres a layer of a composition according to any one of Claims 1 to 44.

**46.** Method according to Claim 45, **characterized in that** the coloration is obtained by applying to the keratin fibres a first base composition containing one or more colouring material precursor(s) in a physiologically acceptable medium, then by applying a composition containing the catalytic system in a physiologically acceptable medium, and vice

versa, the oxidation base(s) being either contained in the base composition or combined with the catalytic system.

**47.** Process according to Claims 45 or 46, **characterized in that** the compositions are applied by spraying.

## Patentansprüche

**1.** Zusammensetzung für das Färben von Keratinfasern, **dadurch gekennzeichnet, dass** sie in einem physiologisch annehmbaren Medium umfasst: eine wirksame Menge wenigstens einer Farbmittel-Vorstufe, die unter Verbindungen ausgewählt ist, die wenigstens einen aromatischen Ring enthalten, der wenigstens zwei Hydroxylgruppen, die von zwei aufeinanderfolgenden Kohlenstoffatomen des aromatischen Rings getragen werden, hat, eine wirksame Menge wenigstens einer Oxidationsbase, die unter den aromatischen Aminen des para- oder ortho-Typs ausgewählt ist, und eine wirksame Menge eines katalytischen Systems, das einen ersten Bestandteil, ausgewählt unter den Mn (II)- und/oder Zn(II)-Salzen und -Oxiden und ihren Gemischen, und einen zweiten Bestandteil, ausgewählt unter den Alkalihydrogencarbonaten, den Erdalkalihydrogencarbonaten und ihren Gemischen, umfasst, wobei die Verhältnisse des ersten und des zweiten Bestandteils wie folgt sind:

$$\frac{[Mn(II)]}{[HCO_3]} \leq 1, \ wobei \ [Mn(II)] \neq 0$$

$$\frac{[Zn(II)]}{[HCO_3]} \leq 1, \ wobei \ [Zn(II)] \neq 0$$

$$\frac{[Mn(II) + Zn(II)]}{[HCO_3]} \leq 1, \ wobei \ [Mn(II)] \ und \ [Zn(II)] \neq 0$$

worin [Mn(II)], [Zn(II)] und [$HCO_3$] jeweils die molaren Konzentrationen an Mn(II), Zn(II) und $HCO_3$ in der Zusammensetzung darstellen.

**2.** Zusammensetzung nach Anspruch 1, **dadurch gekennzeichnet, dass** das Verhältnis $\frac{[Mn(II)]}{[HCO_3]}$ zwischen $10^{-5}$ und $10^{-1}$, vorzugsweise zwischen $10^{-3}$ und $10^{-2}$, und am besten in der Größenordnung von $5 \times 10^{-3}$ liegt.

**3.** Zusammensetzung nach Anspruch 1 oder 2, **dadurch gekennzeichnet, dass** das Verhältnis $\frac{[Zn(II)]}{[HCO_3]}$ zwischen $10^{-4}$ und < 1, vorzugsweise zwischen $10^{-3}$ und < 1, und am besten in der Größenordnung von $5 \times 10^{-1}$ liegt.

**4.** Zusammensetzung nach einem der vorangehenden Ansprüche, **dadurch gekennzeichnet, dass** das Verhältnis $\frac{[Mn(II) + Zn(II)]}{[HCO_3]}$ zwischen $10^{-5}$ und $10^{-1}$, vorzugsweise zwischen $10^{-3}$ und $10^{-2}$ liegt.

**5.** Zusammensetzung nach einem der vorangehenden Ansprüche, **dadurch gekennzeichnet, dass** die Mn(II)- und Zn(II)-Salze unter Chlorid, Fluorid, Jodid, Sulfat, Phosphat, Nitrat, Perchlorat, den Carbonsäuresalzen und ihren Gemischen ausgewählt sind.

**6.** Zusammensetzung nach Anspruch 5, **dadurch gekennzeichnet, dass** das Mn(II)- und/oder Zn(II)-Salz Chlorid ist.

**7.** Zusammensetzung nach Anspruch 5, **dadurch gekennzeichnet, dass** die Carbonsäuresalze Salze von hydroxylierten Carbonsäuren sind.

8. Zusammensetzung nach Anspruch 7, **dadurch gekennzeichnet, dass** das Salz einer hydroxylierten Carbonsäure Gluconat ist.

9. Zusammensetzung nach einem der vorangehenden Ansprüche, **dadurch gekennzeichnet, dass** das Hydrogencarbonat unter Natriumhydrogencarbonat, Kaliumhydrogencarbonat, Magnesiumhydrogencarbonat, Calciumhydrogencarbonat und ihren Gemischen ausgewählt ist.

10. Zusammensetzung nach einem der vorangehenden Ansprüche, **dadurch gekennzeichnet, dass** der aromatische Ring der ersten Farbmittel-Vorstufe, der wenigstens zwei Hydroxylgruppen an aufeinanderfolgenden Kohlenstoffatomen trägt, ein Benzolring oder ein kondensierter aromatischer Ring ist.

11. Zusammensetzung nach Anspruch 10, **dadurch gekennzeichnet, dass** die Farbmittel-Vorstufe eine Verbindung der Formel (I) ist:

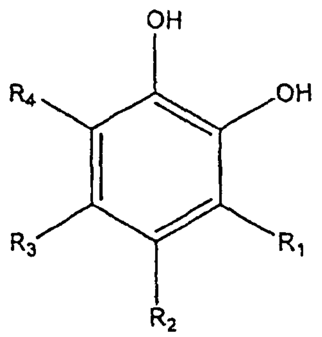

(I)

in der die Substituenten $R_1$ bis $R_4$, die identisch oder unterschiedlich sind, ein Wasserstoffatom, ein Halogenatom, einen Hydroxylrest, einen Carboxylrest, einen Alkylcarboxylatrest, einen gegebenenfalls substituierten Aminorest, einen linearen oder verzweigten, gegebenenfalls substituierten Alkylrest, einen linearen oder verzweigten, gegebenenfalls substituierten Alkenylrest, einen gegebenenfalls substituierten Cycloalkylrest, einen Alkoxyrest, einen Alkoxyalkylrest, einen Alkoxyarylrest, wobei die Arylgruppe gegebenenfalls substituiert sein kann, einen Arylrest, einen substituierten Arylrest, einen gegebenenfalls substituierten heterocyclischen Rest, einen Rest, der gegebenenfalls ein oder mehrere Siliciumatome enthält, darstellen oder zwei der Substituenten $R_1$ bis $R_4$ zusammen einen gesättigten oder ungesättigten Ring bilden können, der gegebenenfalls ein oder mehrere Heteroatome enthält und gegebenenfalls mit einem oder mehreren gesättigten oder ungesättigten Ringen, die gegebenenfalls ein oder mehrere Heteroatome enthalten, kondensiert ist.

12. Zusammensetzung nach einem der Ansprüche 1 bis 10, **dadurch gekennzeichnet, dass** die Farbmittel-Vorstufe unter Flavanolen, Flavonolen, Anthocyanidinen, Anthocyaninen, Hydroxybenzoaten, Flavonen, Iridoiden, wobei diese Verbindungen gegebenenfalls oxyliert und/oder in Form von Oligomeren sein können, Hydroxystilbenen, die gegebenenfalls oxyliert sind, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydroxyindol und seinen Derivaten, 2,3-Dihydroxyindol und seinen Derivaten, Dihydroxycinnamaten, Hydroxycumarinen, Hydroxyisocumarinen, Hydroxycumaronen, Hydroxyisocumaronen, Hydroxychalconen, Hydroxychromonen, Anthocyanen, Chinonen, Hydroxyxanthonen, 1,2-Dihydroxybenzolen, 1,2,4-Trihydroxybenzolen, 1,2,3-Trihydroxybenzolen, 2,4,5-Trihydroxytoluol, 5,6-Dihydroxyindolin und Gemischen aus zwei oder mehreren der vorstehend genannten Verbindungen ausgewählt ist.

13. Zusammensetzung nach einem der Ansprüche 1 bis 10, **dadurch gekennzeichnet, dass** die Farbmittel-Vorstufe unter den Extrakten von Pflanzen, Früchten, Citrusfrüchten, Gemüsen und deren Gemischen ausgewählt ist.

14. Zusammensetzung nach Anspruch 13, **dadurch gekennzeichnet, dass** die Farbmittel-Vorstufe unter den Extrakten von Tee, Traube, Apfel, Banane, Kartoffel und ihren Gemischen ausgewählt ist.

15. Zusammensetzung nach einem der vorangehenden Ansprüche, **dadurch gekennzeichnet, dass** die Farbmittel-Vorstufe in einer Menge von wenigstens 0,001% des Gesamtgewichts der Zusammensetzung vorliegt.

**16.** Zusammensetzung nach einem der vorangehenden Ansprüche, **dadurch gekennzeichnet, dass** die Oxidationsbase unter den ortho- und para-Phenylendiaminen, den Zweifachbasen, den ortho- und para-Aminophenolen, den heterocyclischen Basen sowie den Additionssalzen all dieser Verbindungen mit einer Säure ausgewählt werden kann.

**17.** Zusammensetzung nach Anspruch 16, **dadurch gekennzeichnet, dass** die Oxidationsbase unter den para-Phenylendiaminen der Formel (II) ausgewählt ist:

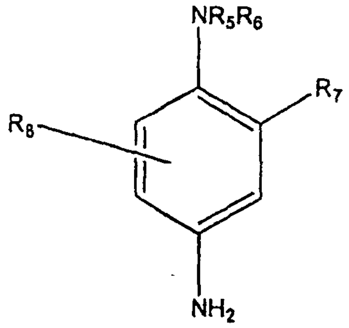

(II)

worin

- $R_5$ ein Wasserstoffatom, einen $C_1$-$C_4$-Alkylrest, einen $C_1$-$C_4$-Monohydroxyalkylrest, einen $C_2$-$C_4$-Polyhydroxyalkylrest, einen ($C_1$-$C_4$) -Alkoxy- ($C_1$-$C_4$) -Alkylrest, einen mit einer stickstoffhaltigen Gruppierung substituierten $C_1$-$C_4$-Alkylrest, einen Phenylrest oder 4'-Aminophenyl darstellt;
- $R_6$ ein Wasserstoffatom, einen $C_1$-$C_4$-Alkylrest, einen $C_1$-$C_4$-Monohydroxyalkylrest oder einen $C_2$-$C_4$-Polyhydroxyalkylrest, einen ($C_1$-$C_4$) -Alkoxy- ($C_1$-$C_4$) -Alkylrest oder einen mit einer stickstoffhaltigen Gruppierung substituierten $C_1$-$C_4$-Alkylrest darstellt;
- $R_7$ ein Wasserstoffatom, ein Halogenatom wie ein Chloratom, einen $C_1$-$C_4$-Alkylrest, einen Sulforest, einen Carboxyrest, einen $C_1$-$C_4$-Monohydroxyalkylrest oder einen $C_1$-$C_4$-Hydroxyalkoxyrest, einen Acetylamino ($C_1$-$C_4$)alkoxyrest, einen Mesylamino($C_1$-$C_4$)alkoxyrest oder einen Carbamoylamino($C_1$-$C_4$)alkoxyrest darstellt;
- $R_8$ ein Wasserstoffatom, ein Halogenatom oder einen $C_1$-$C_4$-Alkylrest darstellt;
- $R_5$ und $R_6$ auch mit dem Stickstoffatom, das sie tragen, einen stickstoffhaltigen Heterocyclus mit 5 oder 6 Kettengliedern bilden können, gegebenenfalls substituiert mit einer oder mehreren Alkyl-, Hydroxy- oder Ureido-Gruppierung(en).

**18.** Zusammensetzung nach Anspruch 17, **dadurch gekennzeichnet, dass** die para-Phenyldiamine unter para-Phenylendiamin, para-Toluylendiamin, 2-Isopropyl-para-phenylendiamin, 2-β-Hydroxyethyl-para-phenylendiamin, 2-β-Hydroxyethyloxy-para-phenylendiamin, 2,6-Dimethyl-para-phenylendiamin, 2,6-Diethyl-para-phenylendiamin, 2,3-Dimethyl-para-phenylendiamin, N,N-Bis-(β-hydroxyethyl)-para-phenylendiamin, 2-Chlor-para-phenylendiamin und ihren Additionssalzen mit einer Säure ausgewählt sind.

**19.** Zusammensetzung nach Anspruch 16, **dadurch gekennzeichnet, dass** die Zweifachbasen aus den Verbindungen, die der Formel (III) entsprechen, ausgewählt sind:

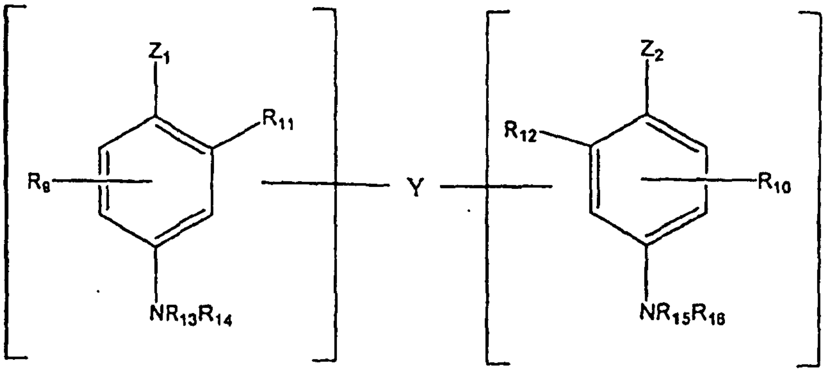

(III)

worin:

- $Z_1$ und $Z_2$, die identisch oder unterschiedlich sind, einen Hydroxylrest oder $-NH_2$ darstellen, die mit einem $C_1$-$C_4$-Alkylrest oder einem Bindungsarm Y substituiert sein können;
- der Bindungsarm Y eine Alkylenkette, die 1 bis 14 Kohlenstoffatome trägt, linear oder verzweigt ist, durch eine oder mehrere stickstoffhaltige Gruppierungen und/oder durch ein oder mehrere Heteroatome wie Sauerstoffatom, Schwefelatom oder Stickstoffatom unterbrochen oder terminiert sein kann und die gegebenenfalls mit einem oder mehreren Hydroxylresten oder $C_1$-$C_6$-Alkylresten substituiert ist;
- $R_9$ und $R_{10}$ ein Wasserstoffatom oder Halogenatom, einen $C_1$-$C_4$-Alkylrest, einen $C_1$-$C_4$-Monohydroxyalkylrest, einen $C_2$-$C_4$-Polyhydroxyalkylrest, einen $C_1$-$C_4$-Aminoalkylrest oder einen Bindungsarm Y darstellen;
- $R_{11}$, $R_{12}$, $R_{13}$, $R_{14}$, $R_{15}$ und $R_{16}$, die identisch oder unterschiedlich sind, ein Wasserstoffatom, einen Bindungsarm Y oder einen $C_1$-$C_4$-Alkylrest darstellen;

wobei es selbstverständlich ist, dass die Verbindungen der Formel (II) nur einen einzigen Bindungsarm Y pro Molekül enthalten.

**20.** Zusammensetzung nach Anspruch 19, **dadurch gekennzeichnet, dass** die Zweifachbasen unter N,N'-Bis-(β-hydroxyethyl)-N,N'-bis-(4'-aminophenyl)-1,3-diaminopropanol, N,N'-Bis-(β-hydroxyethyl)-N,N'-bis-(4'-aminophenyl) ethylendiamin, N,N'-Bis-(4-aminophenyl)tetramethylendiamine, N,N'-Bis-(β-hydroxyethyl)-N,N'-bis-(4-aminophenyl)tetramethylendiamin, N,N'-Bis-(4-methylaminophenyl)tetramethylendiamin, N,N'-Bis-(ethyl)-N,N'-bis-(4'-amino, 3'-methylphenyl)ethylendiamin, 1,8-Bis-(2,5-diaminophenoxy)-3,5-dioxaoctan und ihren Additionssalzen mit einer Säure ausgewählt sind.

**21.** Zusammensetzung nach Anspruch 16, **dadurch gekennzeichnet, dass** die Oxidationsbase unter den para-Aminophenolen der Formel (IV) ausgewählt ist:

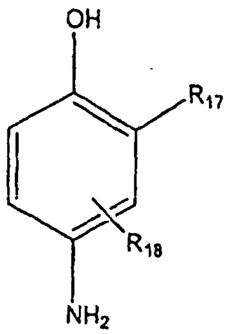

(IV)

worin:

- $R_{17}$ ein Wasserstoffatom, ein Halogenatom wie Fluor, einen $C_1$-$C_4$ -Alkylrest , einen $C_1$-$C_4$-Monohydroxyal-

kylrest, einen ($C_1$-$C_4$)Alkoxy-($C_1$-$C_4$)alkylrest oder einen $C_1$-$C_4$-Aminoalkylrest oder einen Hydroxy-($C_1$-$C_4$)alkylamino($C_1$-$C_4$)alkylrest darstellt;

• $R_{18}$ ein Wasserstoffatom oder ein Halogenatom wie Fluor, einen $C_1$-$C_4$-Alkylrest, einen $C_1$-$C_4$-Monohydroxyalkylrest, einen $C_2$-$C_4$-Polyhydroxyalkylrest, einen $C_1$-$C_4$-Monoalkylrest, einen Cyano($C_1$-$C_4$)alkylrest oder einen ($C_1$-$C_4$) Alkoxy ($C_1$-$C_4$) alkylrest darstellt.

**22.** Zusammensetzung nach Anspruch 21, **dadurch gekennzeichnet, dass** die para-Aminophenole unter para-Aminophenol, 4-Amino-3-methylphenol, 4-Amino-3-fluorphenol, 4-Amino-3-hydroxymethylphenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethylaminomethyl)phenol und ihren Additionssalzen mit einer Säure ausgewählt sind.

**23.** Zusammensetzung nach Anspruch 16, **dadurch gekennzeichnet, dass** die Oxidationsbase ein ortho-Aminophenol ist, das unter 2-Aminophenol, 2-Amino-1-hydroxy-5-methylbenzol, 2-Amino-1-hydroxy-6-methylbenzol, 5-Acetamido-2-aminophenol und ihren Additionssalzen mit einer Säure ausgewählt ist.

**24.** Zusammensetzung nach Anspruch 16, **dadurch gekennzeichnet, dass** die Oxidationsbase eine heterocyclische Base ist, die unter den Pyridinderivaten, Pyrimidinderivaten, Pyrazolinderivaten und deren Additionssalzen mit einer Säure ausgewählt ist.

**25.** Zusammensetzung nach Anspruch 24, **dadurch gekennzeichnet, dass** die Pyridinderivate unter 2,5-Diaminopyridin, 2-(4-Methoxyphenyl)amino-3-aminopyridin, 2,3-Diamino-6-methoxypyridin, 2-(β-Methoxyethyl)amino-3-amino-6-methoxypyridin und 3,4-Diaminopyridin ausgewählt sind.

**26.** Zusammensetzung nach Anspruch 24, **dadurch gekennzeichnet, dass** die Pyrimidinderivate unter 2,4,5,6-Tetraaminopyrimidin, 4-Hydroxy-2,5,6-triaminopyrimidin, 2-Hydroxy-4,5,6-triaminopyrimidin, 2,4-Dihydroxy-5,6-diaminopyrimidin, 2,5,6-Triaminopyrimidin, Pyrazolo[1,5-a]pyrimidin-3,7-diamin, 2,5-Dimethylpyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)(2-hydroxyethyl)amino]ethanol, 2-[(7-Aminopyrazolo[1,5-a]pyrimidin-3-yl) (2-hydroxyethylamino]ethanol, 5,6-Dimethylpyrazolo [1,5-a]pyrimidin-3,7-diamin, 2,6-Dimethylpyrazolo[1,5-a]pyrimidin-3,7-diamin, 2,5,N7,N7-Tetramethylpyrazolo [1,5-a]pyrimidin-3,7-diamin, 3-Amino-5-methyl-7-imidazolylpropylaminopyrazolo[1,5-a]pyrimidin und ihren Additionssalzen und ihren tautomeren Formen, wenn ein tautomeres Gleichgewicht vorliegt, ausgewählt sind.

**27.** Zusammensetzung nach Anspruch 24, **dadurch gekennzeichnet, dass** die Pyrazolderivate aus 4,5-Diamino-1-methylpyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tertbutyl-1-methylpyrazol, 4,5-Diamino-1-tertbutyl-3-methylpyrazol, 4,5-Diamino-1-(β-hydroxyethyl)-3-methylpyrazol, 4,5-Diamino-1-(β-hydroxyethyl)pyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol ausgewählt sind.

**28.** Zusammensetzung nach einem der vorangehenden Ansprüche, **dadurch gekennzeichnet, dass** die Oxidationsbase 0,0005 bis 12 Gew.-% des Gesamtgewichts der Zusammensetzung und vorzugsweise 0,05 bis 8% darstellt.

**29.** Zusammensetzung nach einem der vorangehenden Ansprüche, **dadurch gekennzeichnet, dass** das physiologisch annehmbare Medium ein Solubilisierungsmedium, vorzugsweise mit bakteriostatischen Eigenschaften, ist.

**30.** Zusammensetzung nach einem der vorangehenden Ansprüche, **dadurch gekennzeichnet, dass** das physiologisch annehmbare Medium ein Lösungsmittel oder Lösungsmittelgemisch umfasst.

**31.** Zusammensetzung nach Anspruch 30, **dadurch gekennzeichnet, dass** das Lösungsmittel unter Wasser, Alkoholen, Polyolen, Polyolethern und ihren Gemischen ausgewählt ist.

**32.** Zusammensetzung nach Anspruch 31, **dadurch gekennzeichnet, dass** der Alkohol ein Alkanol oder ein Alkandiol

ist.

**33.** Zusammensetzung nach Anspruch 31 oder 32, **dadurch gekennzeichnet, dass** das Lösungsmittel ein Wasser/Alkohol-Gemisch ist.

**34.** Zusammensetzung nach Anspruch 33, **dadurch gekennzeichnet, dass** der Alkohol bis zu 80 Gew.-% des Gemisches, vorzugsweise 1 bis 50 Gew.-% und am besten 5 bis 20 Gew.-% des Gemisches darstellt.

**35.** Zusammensetzung nach einem der vorangehenden Ansprüche, **dadurch gekennzeichnet, dass** sie einen oder mehrere Koppler, ausgewählt unter den folgenden Verbindungsfamilien, enthält: meta-Aminophenole, meta-Phenylendiamine, meta-Diphenole, Naphthole und die heterocyclischen Koppler wie z.B. Indolderivate, Indolinderivate, Sesamol und seine Derivate, Pyridinderivate, Pyrazolotriazolderivate, Pyrazolone, Indazole, Benzimidazole, Benzothiazole, Benzoxazole, 1,3-Benzodioxole, Chinoline und ihre Additionssalze mit einer Säure.

**36.** Zusammensetzung nach Anspruch 35, **dadurch gekennzeichnet, dass** die Koppler unter 2,4-Diamino-1-(β-hydroxyethyloxy)benzol, 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2-Amino-4-(β-hydroxyethylamino)-1-methoxybenzol, 1,3-Diaminobenzol, 1,3-Bis-(2,4-diaminophenoxy)propan, Sesamol, 1-Amino-2-methoxy-4,5-methylendioxybenzol, α-Naphthol, 6-Hydroxyindol, 4-Hydroxyindol, 4-Hydroxy-N-methylindol, 6-Hydroxyindolin, 2,6-Dihydroxy-4-methylpyridin, 1H-3-Methylpyrazol-5-on, 1-Phenyl-3-methylpyrazol-5-on, 2-Amino-3-hydroxypyridin, 3,6-Dimethylpyrazolo[3,2-c]-1,2,4-triazol, 2,6-Dimethylpyrazolo[1,5-b]-1,2,4-triazol und ihren Additionssalzen mit einer Säure ausgewählt sind.

**37.** Zusammensetzung nach Anspruch 35 oder 36, **dadurch gekennzeichnet, dass** der Koppler oder die Koppler 0,0001 bis 15 Gew.-% des Gesamtgewichts der Zusammensetzung und vorzugsweise 0,001 bis 10% darstellt/darstellen.

**38.** Zusammensetzung nach einem der vorangehenden Ansprüche" **dadurch gekennzeichnet, dass** sie frei von jedem Chelatbildner für das Salz von Mn(II) und/oder Zn(II) ist.

**39.** Zusammensetzung nach einem der vorangehenden Ansprüche, **dadurch gekennzeichnet, dass** sie in Form eines Aerosols oder eines Pumpsystems ohne Luftaufnahme formuliert ist.

**40.** Zusammensetzung nach einem der Ansprüche 1 bis 38, **dadurch gekennzeichnet, dass** sie sich in Form von zwei getrennten Bestandteilen präsentiert, wobei ein erster Bestandteil das katalytische System und die Oxidationsbase in einem physiologisch annehmbaren Medium gelöst enthält und ein zweiter Bestandteil die Farbmittel-Vorstufe in einem physiologisch annehmbaren Medium gelöst enthält.

**41.** Zusammensetzung nach einem der Ansprüche 1 bis 38, **dadurch gekennzeichnet, dass** sie sich in Form von zwei getrennten Bestandteilen präsentiert, wobei ein erster Bestandteil das katalytische System in einem physiologisch annehmbaren Medium gelöst enthält und ein zweiter Bestandteil die Farbstoffvorstufe und die Oxidationsbase in einem physiologisch annehmbaren Medium gelöst enthält.

**42.** Zusammensetzung nach Anspruch 40 oder 41, **dadurch gekennzeichnet, dass** sie in einem Pumpsystem mit zwei getrennten Kammern formuliert ist, wobei die Farbmittel-Vorstufe(n) in einer Kammer ist/sind und das katalytische System in der anderen Kammer ist und die Oxidationsbase(n) in der einen oder der anderen der zwei Kammern ist/sind.

**43.** Zusammensetzung nach einem der Ansprüche 1 bis 38, **dadurch gekennzeichnet, dass** sie in Form eines Briefchens oder mehrerer Briefchen, das/die für Sauerstoff undurchlässig ist/sind, formuliert ist.

**44.** Zusammensetzung nach Anspruch 43, **dadurch gekennzeichnet, dass** sie in Form von zwei für Sauerstoff undurchlässigen Briefchen formuliert ist:

- ein erstes Briefchen, das das katalytische System und die Oxidationsbase(n) enthält und ein zweites Briefchen, das die Farbmittel-Vorstufe enthält, oder
- ein erstes Briefchen, das das katalytische System enthält und ein zweites Briefchen, das den oder die Farbmittel-Vorstufe(n) und die Oxidationsbase(n) enthält.

**45.** Verfahren zum Färben von Keratinfasern, **dadurch gekennzeichnet, dass** es aus einem Auftragen einer Schicht einer Zusammensetzung nach einem der Ansprüche 1 bis 44 auf die Haut und/oder Keratinfasern besteht.

**46.** Verfahren nach Anspruch 45, **dadurch gekennzeichnet, dass** das Färben erreicht wird, indem eine erste Grundzusammensetzung, die eine oder mehrere Farbmittel-Vorstufe(n) in einem physiologisch annehmbaren Medium enthält, auf die Keratinfasern aufgetragen wird, dann eine Zusammensetzung, die das katalytische System in einem physiologisch annehmbaren Medium enthält, aufgetragen wird, oder dass umgekehrt die Oxidationsbase(n) entweder in der Grundzusammensetzung enthalten ist/sind oder mit dem katalytischen System kombiniert ist/sind.

**47.** Verfahren nach Anspruch 45 oder 46, **dadurch gekennzeichnet, dass** die Zusammensetzungen durch Pulverisieren angewendet werden.